Europäisches Patentamt

European Patent Office

Office européen des brevets

(19)

(11) Publication number: **0 398 663**
**A1**

# EUROPEAN PATENT APPLICATION

(21) Application number: **90305245.4**

(22) Date of filing: **15.05.90**

(51) Int. Cl.5: **A61M 31/00, A61D 7/00**

(30) Priority: **16.05.89 GB 8915002**

(43) Date of publication of application:
**22.11.90 Bulletin 90/47**

(84) Designated Contracting States:
**AT BE CH DE DK ES FR GB GR IT LI LU NL SE**

(71) Applicant: **COOPERS ANIMAL HEALTH LIMITED**
**Berkhamsted Hill**
**Berkhamsted Hertfordshire HP4 2QE(GB)**

(72) Inventor: **Shepherd, Michael Trevor**
**Coopers Animal Health Limited,**
**Berkhamsted Hill**
**Berkhamsted, Hertfordshire(GB)**
Inventor: **Stratford, Michael George**
**Coopers Animal Health Limited,**
**Berkhamsted Hill**
**Berkhamsted, Hertfordshire(GB)**

(74) Representative: **Matthews, Derek Peter et al**
**Frank B. Dehn & Co. Imperial House 15-19**
**Kingsway**
**London WC2B 6UZ(GB)**

(54) **Dispensing devices.**

(57) An intraruminal device for administering an active agent into the rumen of an animal, comprising a chamber (3) enclosing the active material between a closure (2) and propulsion means (a piston) (8), one of the closure and propulsion means has one or more apertures for expulsion of the active material.

The active material is conveniently expelled from the device by means of a piston biased against it by the tension of the spring. The tension spring may be constructed of materials that are environmentally preferred to metal springs and in addition hold the device together.

*FIG. 2*

EP 0 398 663 A1

The present invention relates to devices for dispensing active materials into an environment over a long period at a predetermined rate. More specifically, it relates to devices for extruding an active material into the rumen of a ruminant.

European Patent Application 149510 describes an intra rumenal bolus comprising a hollow chamber sealed at one end and having a closure provided with a small aperture at the other end and containing a matrix of active material. The bolus also contains a piston which is biased by means of a compression spring against the matrix of biologically active material thereby keeping the latter in contact with the aperture. The rumenal fluids dissolve the leading edge of the matrix and the bias provided by the sprung piston ensures that the dissolved biologically active material is expelled from the bolus. The bolus is provided with a weight of sufficient density to prevent the animal from regurgitating the bolus.

However, such devices suffer from the disadvantage that once the biologically active material is dispensed, there is still residual energy left in the spring which can act to push the device apart. It has now been discovered that the metal compression spring of this device can be replaced by a tension spring. This tension spring may be constructed of materials that are environmentally preferable to metal and can additionally assist in maintaining the integrity of the device.

Accordingly, the present invention provides a device for dispensing active materials into an environment, comprising a chamber enclosing the active material contained between a closure and propulsion means, one of the closure means and propulsion means being provided with one or more apertures for release of the active material characterised in that the device comprises a tension spring biasing the propulsion means towards said closure. Optionally both the propulsion means and the closure contain one or more apertures for expulsion of the active material. The active material may be biologically or chemically active.

Conveniently the device is provided with means for retaining the device in the environment.

Suitably the device is an intra-rumenal bolus.

Suitably the bolus comprises a tubular casing made of plastic or other rigid non-deformable material that retains its integrity until all the material has been expelled from the device into the environment, for example the reticulo-ruminal sac of a ruminant. The casing may have one or two separable or fixed end closures and optionally may have an internal chamber or chambers for active material and optionally an internal weight or means for retention in the environment determined by the external shape.

Conveniently the active material is released from the device by means of a piston (the propulsion means) biased against it by the tension of the spring. Conveniently the piston forms a closure to the chamber containing the active material. Conveniently the piston is provided by the weight. The spring may be a coiled spring conveniently of metal or plastic construction or may be of one or more strips, bands or sheaths of elastomeric, preferably rubber material. A spring may be attached at one end to the casing adjacent to the closure or to the closure itself, and the second end attached to a suitable second part of the device to provide tension to the spring, one of the points of attachment being mobile. Preferably the mobile attachment is the following edge of the weight or the propulsion means for the active material. The spring may be located in a hollow in the casing wall or in an aperture between the casing and the active material or passing through the active material. A coiled spring may be provided about the active material and located within the casing.

Suitably the means for retaining the device in the environment is a weight, which in the case of a bolus will provide sufficient density to prevent the device being regurgitated. The weight may be a single piece of a sufficiently dense material such as metal or dense filler which is advantageously degraded by the environment after the active material is expended, or it may be in the form of shot constrained to remain in the device, for example iron shot dispersed in a degradable base material or held in a retaining sleeve of an insoluble plastics material. Additionally or alternatively the device may be retained in the environment by adopting a geometric configuration which prevents its expulsion from the environment, for example by adopting a geometric configuration in the rumen that obstructs its passage up the animals oesophagus. Devices which may be meat works compatible preferably comprise an unsintered metal weight, for example of the type described in U.K. Application No. 8802099.

Preferably the weight is substantially cylindrical and is located at one end of the device. The outermost face of the weight is conveniently domed or flat.

In one embodiment the weight is cylindrical and provides a closure for the chamber containing active material. If the weight is made of unsintered metal it may be encased in or be supported by an insoluble material having a suitable attachment to the casing. Preferably there is provided a seal such as a washer between the weight and the cylinder which seals the chamber containing the biologically active material.

The weight may have an external shape adapted to cooperate externally with the tension spring or springs. Suitably it may be dome-ended and located in the closed end of a rubber sheath, the open end of which is attached under tension to the cylinder casing or compartment wall.

In one embodiment the weight has one or more apertures parallel to or coincident with its axis which are adapted to receive one or more tension springs. Conveniently the apertures are adapted to align with similar apertures in the active material.

In a further embodiment the cylinder wall is spaced apart from the edge of the chamber contents between the points of attachment of each spring, and the space is adapted to receive the coiled, strip, band or sheath-like tension spring.

In a further embodiment, the cylinder wall comprises elongate hollows having suitable points of attachment adapted to receive the spring, as described above.

In one embodiment, means for securing the ends of the spring or springs to the casing or closures include projections of the casing or closures, or of plates or bars abutting the following edge of a closure. Two suitably aligned projections from a fixed and a mobile point of attachment are adapted to tension a band which is looped between the two projections or to tension a strip by engaging eyes moulded in its ends, or a coil by engaging hooks formed at its ends.

In a further embodiment a coiled spring projects inwardly towards its axis at each end, the projections adapted to engage in apertures sunk in the outermost face or in the sides of closures. There may be one circumferal recess in the side of the closure adapted to engage one or more springs.

In a further embodiment the strip or coiled spring may be a moulded extension of the closures.

In a further embodiment a rubber sheath attaches at its open end to the rim of the cylinder. Suitably the sheath is adapted to extend over the rim and fold back against the outside wall of the cylinder, and is engaged against the cylinder wall by its own tension or by a tension garter placed about its rim. Optionally the sheath has a moulded rim which engages in a circumferal recess about the cylinder wall.

Conveniently one or both of the closure means and the propulsion means are provided by one or two weights. Conveniently the propulsion means is a piston which expels the active material, and is moveable. Optionally both the closure means and the propulsion means are moveable.

In one embodiment, one weight provides a piston closure to the chamber containing active material and is moveable. The weight attaches to the spring suitably as disclosed above. The weight is of suitable cross-section to drive into the chamber towards the closure as the active material is expelled from the device at the closure, and prevents the development of vacuum or partial pressure within the device.

Alternatively the biologically active material is expelled from apertures in the mobile weight as the weight is drawn towards the closure.

The cylinder, closure and weight casing may be made from a plastics material such as thermoplastic.

The devices of the present invention are intended for oral administration to ruminants and their dimensions will be such as to allow them to pass freely down the animal's oesophagus into the animal's rumeno-reticular sac whilst being of sufficient size to enable incorporation of adequate quantities of active agent. Thus, convenient dimensions of devices for administration to cattle are between 10 and 45mm in diameter, typically 25mm in diameter, and 20 and 150mm in length, typically 100mm in length, whilst those for sheep are between 5 and 30 mm diameter, typically 20mm, and 10 and 100mm, typically 45mm, length.

Suitably the active material is a biologically active material in the form of a solid matrix which is dissolvable in the rumenal fluids. Thus, the rate of release of biologically active material from the bolus is dependant upon the solubility of the matrix in the rumenal fluids and other factors such as spring tension, viscosity and the number and diameter of the aperture in the end closure. The biologically active material is suitably distributed in a solid matrix formed from one or more tablets or blocks. If it is required to dispense the material discontinuously then successive tablets or blocks of matrix with and without biologically active material may be assembled and incorporated in the device.

The active agents for incorporation in the devices of the present invention may be selected from therapeutic agents, growth promotants, vaccine formulations, nutrients, agents affecting fertility and other substances necessary for the physical well being of the ruminant.

Examples of therapeutic agents for in vivo use include anti-infectives, e.g., an antiviral agent such as acyclovir, idoxviridine or vidarabine; anti-bacterial agents such as penicillins, tetracyclines, erythromycin, neomycin, polymyxin B, gentamycin, nystatin, benzylpyrimidines such as trimethoprim or baquiloprim optionally in combination with a sulphonamide such as sulphadiazine, sulphadimidine, sulphadoxine or sulphadimethoxine, or bacitracin; and anti-protozoals such as anti-coccidials.

Active agents for use in the bolus of the present invention also include anti-parasitic agents such as anthelmintics, for example oxfendazole (i.e. 2-methoxycarbonylamino-5-phenylsulphinylbenzimidazole), flubendazole, oxibendazole, parbendazole, niridazole, mebendazole, fenbendazole, cambendazole, albendazole, metronidazole, thiabendazole, levamisole, tetramisole, closantel, bromoxanide, rafoxanide, clioxanide, oxyclozanide, salantel, morantel, resorantel, pyrantel, praziquantel, febantel, oxantel, carbantel, piperazine, nicolosamide, brotianide, thiophanate, bephenium, pyrvinium, diethylcarbamazine, suramin, dichlorophen, paromomycin, stibophen, antimony sodium dimercaptosuccinate, hycanthone, metrifonate, antimony barium tartrate, antimony potassium tartrate, chloroquine, emethine, bithionol, hexylresorcinol, tetrachloroethylene, mirasan, miracil, lucanthone, furapromidium, oxamniquine, tubercidin, amphotalide, nicarbazin, Hetol (Trade Name), Hetolin (Trade Name), nitroxynil, disophenol, Bitin-S (Trade Name), bromofenophos, menichlopolan, thiosalicylanilide, diamphenethide, bunamidine, bitoscanate, nitroscanate, amoscanate, diuredosan, arsenamide, thiazothienol, thiazothielite, haloxon, dithiazanide iodide, bidimazium iodide, methyridine dymanthine trichlabendazole, chlorsulan and avermectins such as ivermectin.

Nutrients or other substances conducive to physical health, for use in the bolus according to the present invention, include vitamins and agents which have a vitamin-like physiological role. Vitamin mixes, particularly containing one or more B vitamins, may be included for the treatment of fodder poisoning or acute bacterial toxaemia in animals. For the assistance of rehydration in fluid-electrolyte loss associated with scours in calves, lambs and pigs, the biologically active material in the bolus of the invention may comprise a composition of suitable electrolytes including sodium chloride, and/or one or more appropriate sugars, for example glucose. Particularly when intended for administration to grazing animals, such nutrients may include trace elements, for example selected from magnesium, zinc, copper, cobalt and selenium.

When the device contains a degradable metal this is conveniently selected from magnesium, aluminium, zinc, titanium and their alloys, an alloy of magnesium and one or more other metals is preferred, for example selected from the following (all percentages being by weight): silicon up to 1.5%, nickel up to 1%, zinc up to 15% (for example about 12%), manganese up to 2%, copper up to 4% (for example about 2% or about 3%), aluminium up to 15% (for example about 12%), and zirconium up to 0.8%. It will be appreciated by those skilled in the art that not all of the above metals are mutually compatible.

Other active agents for use in the device of the present invention include growth promotants such as tetronasin, lasalocid, monensin, lysocellin, virginiamycin, zinc bacitracin, flavomycin, avoparcin, nitrovin, thiopeptin, halquinol, carbadox, tylosin and arsanilic acid (including carbarsone) or combinations of these. Some of these agents (e.g. zinc bacitracin) are also antibacterial agents.

For enhancing the fertility of animals or inducing ovulation, one or more appropriate hormone or hormone mimetics such as prostalene, optionally in combination with vitamin E, may be included.

In particular, therapeutic agents may be provided in discrete units such as capsules, cachets or tablets, each containing a predetermined amount of agent, as a powder or granules. Such formulations may be prepared by any of the methods of pharmacy and may include the step of bringing into association the agent with a binder and/or carrier which constitutes one or more accessory ingredients. In general they are prepared by uniformly and intimately admixing the agent with liquid or finely divided solid carriers or both, and then, if necessary, shaping the product into the desired presentation. For example, a tablet may be prepared by compression or moulding a powder or granules of the agent, optionally with one or more accessory ingredients. Compressed tablets may be prepared by compressing, in a suitable machine, the agent in a freeflowing form such as a powder or granules optionally mixed with a binder, lubricant, inert diluent, surface active or dispersing agent(s). Moulded tablets may be made by moulding, in a suitable machine, the powdered agent moistened with an inert liquid diluent. Alternatively, such formulation may be prepared by admixture of the agent with one or more of the conventional solid carriers, for example cocoa butter, and shaping of the resulting mixture.

When the therapeutic agent is formulated as a tablet, the tablet may have, for example, especially for administration to cattle, a weight in the range of 0.5 to 5g, for example 0.75 to 2g, typically about 1g. When the tablet is provided with one aperture parallel to or coincident with its axis, typically this may have a diameter in the range of from 4 to 15mm. The weight of active ingredient in the tablet may for example be in the range of from 50 to 95% w/w, typically about 75% w/w. Suitable binders, carriers and other diluents include disintegrants such as starch, e.g. 5 to 15%, typically 10% w/w, binders such as polyvinylpyrrolidene (PVP), e.g. 0.5 to 5% w/w, typically 2.5% w/w and lubricants such as magnesium stearate, e.g. 0.2 to 2.5% w/w, typically 1.0% w/w. In any tablet formulation, if desired, the remaining bulk of the tablet may be made up with a material such as lactose. It will be appreciated that such compositions of therapeutic agent may also be formulated as a cylinder when intended for use in a bolus having a single or multiple segments of therapeutic agent. Such a cylinder may have a weight of e.g. 5 to 100g, typically about 80g, a diameter from 4 to 35mm, typically about 22mm and an overall length of e.g. 20 to 150 mm, typically about 100mm. The weight may also be incorporated as a discrete mass within the drug matrix. Such a matrix conveniently comprises, monostearin, carnuba wax and barite and may have a weight of e.g. 10 to 300g, typically about 100g for cattle, a diameter of 10 to 45mm, typically about 22mm for cattle, and an overall length of e.g. 10 to 150mm, typically about 100mm for cattle.

The boluses of the present invention will be assembled by standard production techniques, e.g. forming the casing by standard techniques, such as thermoforming, and loading the weight and therapeutic agents (see Schemes 1 and 2) and sealing the bolus by conventional means, e.g. transfer moulding, insert moulding or injection moulding.

The boluses are conveniently administered by means of an oesophageal balling gun.

The present invention will now be described in more detail by way of the following exemplary embodiments and with reference to accompanying drawings in which the device is an intra-rumenal bolus and the environment is the rumen of the ruminant:

Figure 1 represents a longitudinal section of a device of the invention;

Figure 2 represents a longitudinal section of a device of the invention illustrating a further embodiment of the spring means;

Figure 3 represents an end elevation of the device of figure 2 showing the closure and apertures;

Figure 4 represents a longitudinal section of a device of the invention comprising a further embodiment of the composition of the biologically active material;

Figure 5 represents a longitudinal section of the device of Figure 2 after the biologically active material has been expelled;

Figures 6 & 8 represent longitudinal sections of a device of the invention comprising two further embodiments of the spring means;

Figure 7 represents a cross section through X-X of the device of Figure 6;

Figure 9 represents a longitudinal section of a device of the invention comprising a further embodiment of the extrusion means;

Figure 10 represents a longitudinal section of the device of Figure 9 comprising a modification of the extrusion means;

Figures 11 & 12 represent longitudinal sections of a device of the invention comprising a further embodiment of the retention means;

Schemes 1 and 2 illustrate the loading technique for assembly of devices of the invention.

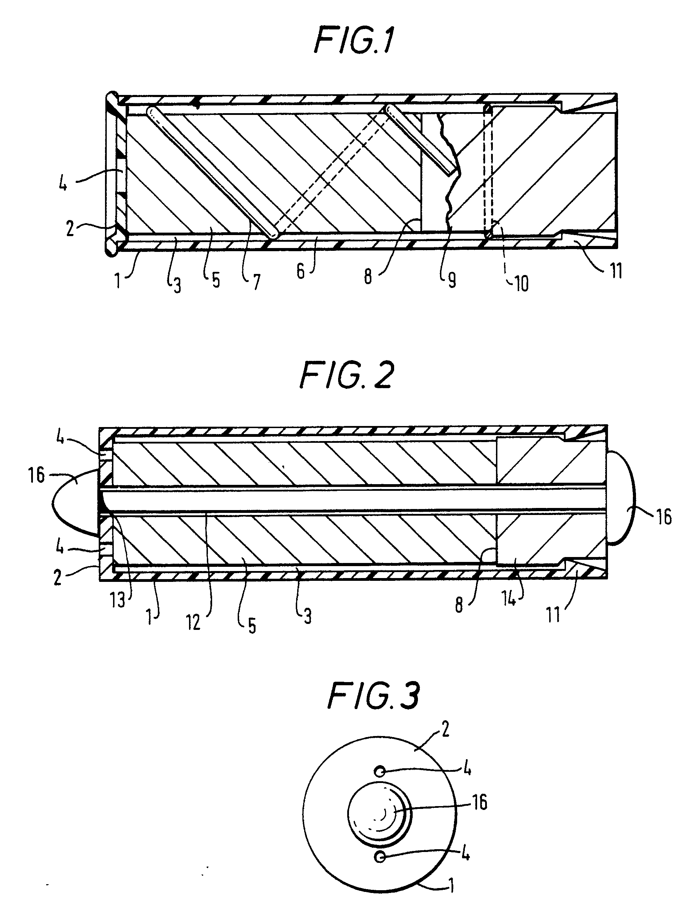

The devices of Figures 1 to 12 comprise a cylinder (1) which is hollow and has a closure (2) at its first end, the cylinder and closure both being constructed of a rigid plastics material that is insoluble or soluble or degradable over a long period in the environment in which the device is to be employed. The cylinder provides a chamber (3) closed at both ends, which has access apertures (4) and which contains tabletted biologically active material (5) abutting both closures.

Referring to Figure 1, the chamber (3) provided at the first end of the cylinder is of uniform internal cross-section and has a first closure (2) in which an aperture (4) provides means for extrusion of the active material (5) into the environment. The volume and composition of tablet (5) determines the amount and nature of the dose to be administered. The tablet is centrally positioned in the chamber (3) and is of a uniform diameter such that its edge is spaced apart from the chamber walls to provide a cavity (6). A tension spring (7) is located in the cavity (6) spiralled about the active material (5) and tensions a piston (8) against the following end of the active material, one end of the spring being attached to the piston, its second end being attached to the chamber wall at the leading edge of the active material. The piston (8) provides a mobile second closure to the chamber (3) causing rumen fluid to be drawn into the open end of the cylinder behind the piston preventing development of pressure differences within the device. The piston (8) is provided by one end of a cylindrical mobile weight (9) formed from a shot filled insoluble sleeve. A seal (10) projecting inwardly from the cylinder wall (1) effects a sliding seal at the second closure of chamber (3) with the edge of the mobile weight (9), preventing undesired leakage of the rumen fluid into the chamber (3). An inwardly projecting flange (11) prevents expulsion of the weight from the device in the event that the spring is dislocated or ruptured, thus retaining the spring and any remaining active material within the device.

In a further embodiment of the device of Figure 1, the active material is present in the form of two or more separate tablets(5) which are stacked centrally in chamber (3).

In a further embodiment of the device of Figure 1, the weight (9) comprises material degradable in the environment, contained within a plastic insoluble sleeve.

Referring to Figure 2, an alternative embodiment of the invention is illustrated wherein the spring means is provided by a rubber ligature (12). The closure (2) of the cylinder (1) has two apertures (4) for extrusion of the active material and a central aperture (13) at which is secured one end of the ligature (12). The cylinder contains a mobile annular weight (14) and one or more annular tablets (5) of active material. The ligature (12) is located in the central aperture provided by the annulus such that the second end of the ligature (12) secures to the following end of piston (8) provided by the weight (14) thereby tensioning the ligature. The ligature is secured at its ends by means of moulded lugs (16) which impinge on the outsides of the closure (2) and following end of the weight (14).

In Figure 3, the device of Figure 2 is seen in end elevation, illustrating the extrusion apertures (4) positioned about the secured lug-end (16) of the axial ligature (12).

In a further embodiment, illustrated in Scheme 2, the spring means comprises a rubber "band" passing through an "eye" on a separate rubber or plastic lug end (16), and hooked onto an elongate peg-end which is impinged on the outside of the following end of the weight (14).

In a further embodiment of the invention, the device of Figure (2) provides a mobile annular weight (14), which is meat-works compatible made of green metal contained in an insoluble plastic sleeve or casing.

In a further embodiment of the sealing means for the chamber of the device, there is provided a sealing washer, located between the adjacent ends of the active material (5) and weight (14), which is drawn along the cylinder by the piston (8) effecting a sliding seal for chamber (3).

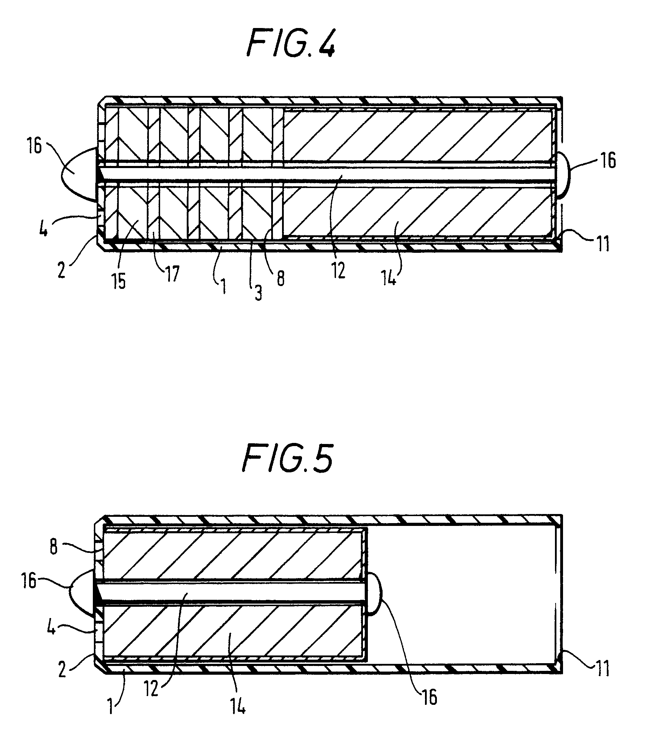

Figure 4 illustrates an alternative embodiment of the device of Figure 2 wherein the annular tablets of active material (15) are alternated with annular tablets of inert material (17). In this embodiment, the device is intended for pulse release administration of the active material into the environment.

Figure 5 illustrates the device of Figure 4 after the active material (15) has been expended. The residual tension of the ligature (12) and the flange (11) prevent the components of the device from being expelled into the environment.

The device of Figure 1, after the active material (5) has been expelled similarly retains the spring (7) preventing any adverse effects caused by its release into the environment. Should the spring (7) be released it reverts advantageously to the contracted state, wherein it is least likely to damage or entangle its environment.

Figure 5 illustrates the weight contacting the closure (2). The shot content of the weight (9) is released through the aperture (4) of closure (2) thereby reducing the density of the device, which is then regurgitated. The alternative embodiment device of Figure 1, wherein the weight (9) comprises degradable metal, is regurgitated after seepage of rumen fluid through aperture (4) degrades the weight. The alternative embodiment device of Figure 2 is meat-works compatible and need not be regurgitated.

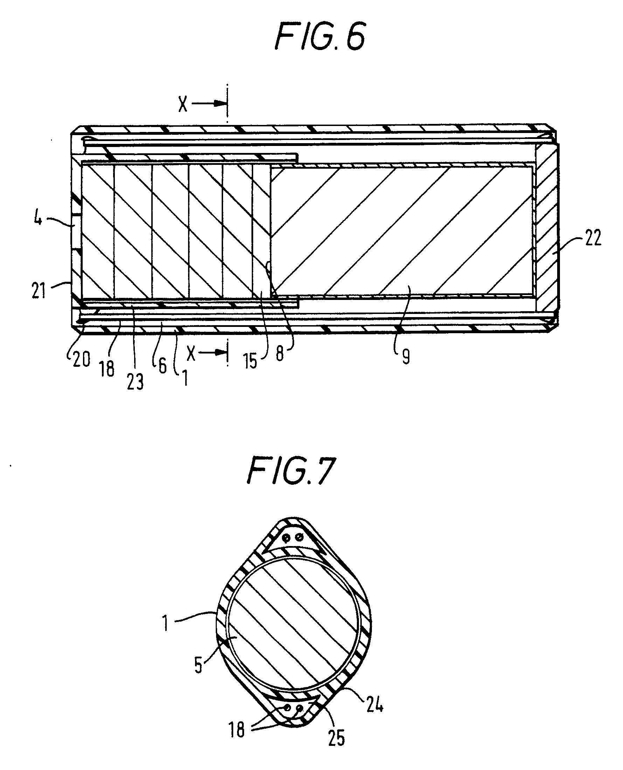

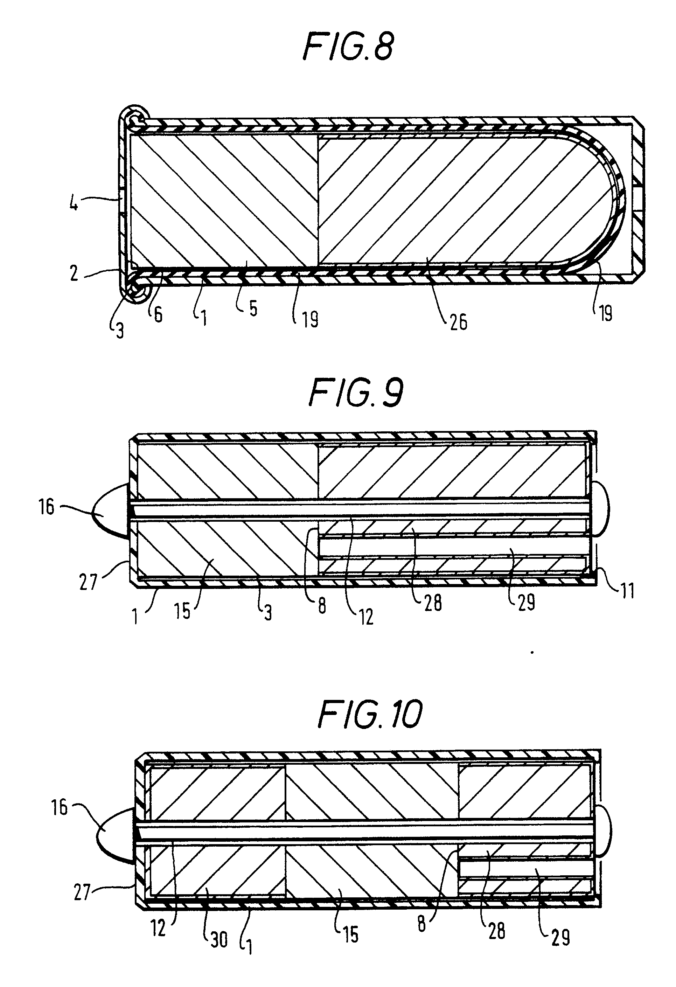

Figures 6 and 8 illustrate further alternative embodiments of the invention wherein the device is operated by two or more rubber ligatures (18) or by a rubber sheath (19). In Figure 6, the ligatures (18) are located in a cavity (6) between the cylinder wall (1) of the device and the active material (5). The ends of the ligatures (18) are moulded loops, hooked onto projections (20) protruding at right angles to the axis of the device from the closure (21) and from a plate (22) abutting the following end of the weight. Closure (21) has a cylindrical flange (23) which extends perpendicularly to provide chamber (3) containing the active material (5) and overlaps with the sleeve of the weight (9) to seal the chamber at its end closure. The cylindrical flange (23) and the cylinder (1) wall are spaced apart to provide the cavity (6) in which are located the ligatures (18).

In a further embodiment of the invention, the device of Figure 6 has a uniformly non-cylindrical exterior, its wall bowing in two or more places from the cylindrical. The interior of the wall is cylindrical, a cavity within the cylinder wall thus existing at each bow (24) of the external wall. The cavities (25) are elongate along the length of the device and contain the ligatures (18).

In yet another embodiment of the device of Figure 6 the ligatures (18) are rubber loops (i.e. rubber "bands").

Figure 7 illustrates in section the arrangement of the cylinder wall (1) having bowed exterior (24) providing elongative cavities (25) containing ligatures (18).

Figure 8 illustrates a seventh embodiment of the invention wherein the device is operated by a rubber sheath (19) attached along its open end to the circumference of the cylinder wall (1) in conjunction with the closure (2). The sheath contains the active material (5) and a dome-ended weight (26) such that the sheath tensions the weight (26) and closure (2) against the active material (3). The device embodies features of the abovementioned alternative devices.

Figure 9 illustrates a further embodiment of the extrusion means of the invention wherein the device of Figure 2 has a sealed closure (27) and the mobile weight (28) has one or more extrusion apertures (29) along its length. In a first version of the embodiment, the aperture or apertures (29) are typically displaced from the centre about the aperture containing the ligature (12) which is axially aligned.

In a second version, the devices of Figures 1 and 6 employ this means of extrusion through the mobile weight, which provides one extrusion aperture which is axially located.

Figure 10 illustrates a further variation of this embodiment of the invention wherein the device of Figure 9 has a first weight (28) abutting one edge of the active material and having one or more extrusion apertures (29) and a second non-mobile, cylindrical weight (30) abutting the other edge of the active material tablets (15).

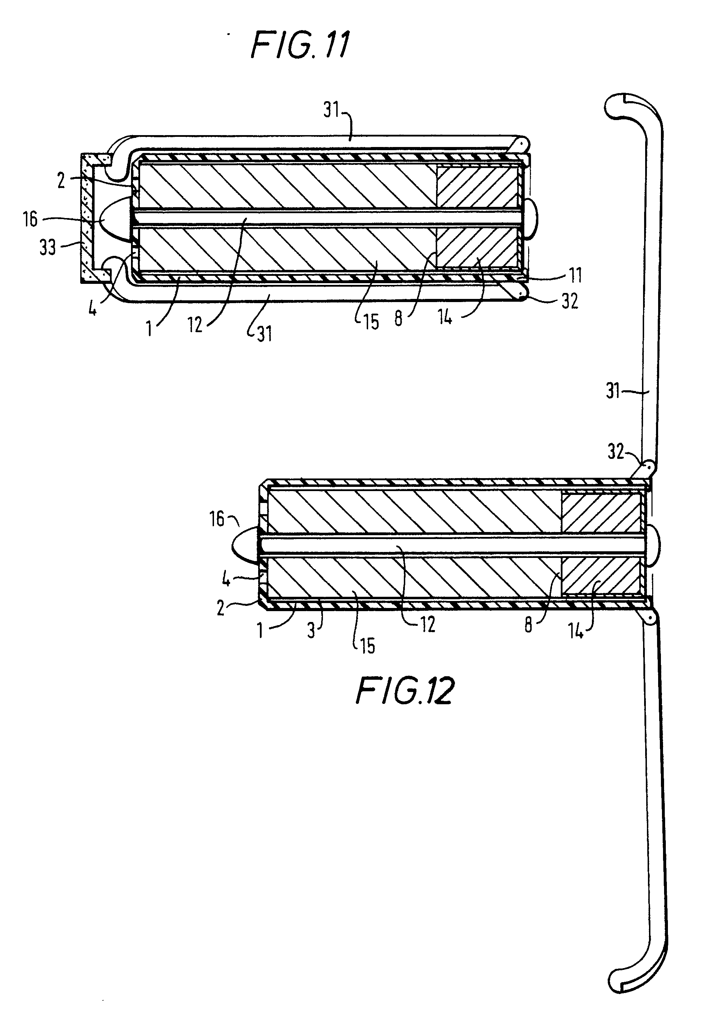

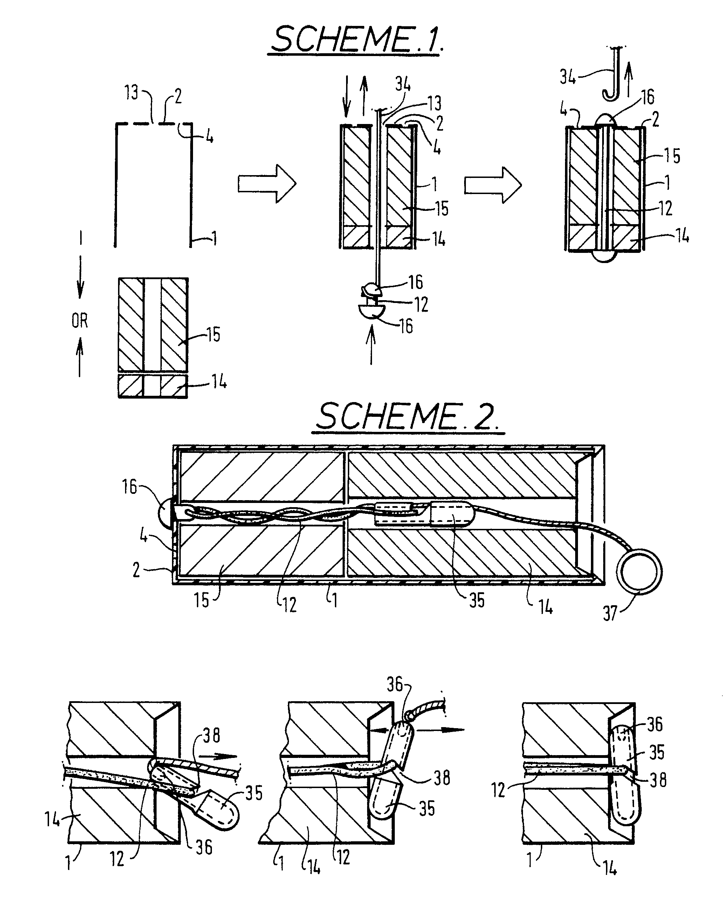

There is provided a further embodiment of the invention as illustrated in Figures 11 and 12, wherein a device of the invention is retained in the environment by its external configuration. A shortened device of Figure 2 is provided with a smaller weight (14) and two retention wings (31) which are attached to the casing of the device by means of spring hinges (32) such that the wings are constrained to lie against the walls of the cylinder by a soluble cap (33) made from a biologically degradable material such as Polyox or Gelatin etc (Figure 11). The device is then easily introduced by conventional means into the rumen where the cap (33) is dissolved to release the wings which adopt a configuration at right angles to the axis of the device (Figure 12). The cap (33) prevents the device from being sprung open whilst in the oesophagus of the ruminant.

This embodiment may be incorporated in other devices of the invention such as those described above.

The technique for tensioning individually loaded devices of the invention is illustrated in Schemes 1 and 2.

Scheme 1 relates to the device of Figure 2, wherein the active material tablet or tablets (15) followed by the weight (14) are loaded into the cylinder (1) having moulded closure (2). A hooked rod (34) is passed through the apperture and hooked onto one lug-end (16) of the ligature (12). The ligature is drawn into the annulus, its free lug-end impinging on the outside of weight (14), and its hooked lug-end impinging as it is released from the rod on the outside of the closure (2), thus tensioning the ligature (12).

Scheme 2 relates to the device of Figure 2 wherein the ligature (12) is provided by a rubber "band" passing through an "eye" in the lug-end, as described above. The device is loaded as in Scheme 1. An elongate peg (35) has a cavity at one end (36) into which is slipped the bulbous end of a cord passing through the central aperture of the device and attached to a pull-ring (37). The peg has a cleft (38) at its centre and angled toward the cavity (36). The rubber "band" is hooked onto the cleft (38), and the peg drawn through the device by pull-ring (37). As the peg (35) emerges from the aperture, it pivots about its centre, releasing the cord, and impinging on the outside of the weight, thereby tensioning the rubber "band".

## Claims

(1) A device for dispensing active materials into an environment, comprising a chamber enclosing the active material contained between a closure and a propulsion means, at least one of the closure and propulsion means being provided with one or more apertures for release of the active material characterised in that the device comprises a tension spring biasing the propulsion means towards said closure.

(2) A device according to claim 1 wherein the propulsion means is a piston.

(3) A device according to claim 1 and 2 wherein the tension spring is a coiled spring.

(4) A device according to claim 1 and 2 wherein the tension spring is one or more strips, bands or sheaths of elastomeric material.

(5) A device according to any one of claims 1 to 4 wherein means are provided for retaining the device in the environment.

(6) A device according to claim 5 wherein the retention means comprises of a weight attached to the device.

(7) A device according to claim 5 wherein the retention means comprises of wings attached to the chamber movable between a compact position parrallel to the side of the chamber to a position approximately 90° to the chamber.

(8) A device according to any one of claims 1 to 7 charged with a biologically active material for administration to a ruminant.

(9) A device according to claim 8 wherein the biologically active material may be selected from therapeutic agents, growth promotants, vaccine formulations, nutrients, agents affecting fertility and other substances necessary for the physical well being of the ruminant.

(10) A device according to claim 8 wherein the biologically active material is tetronasin, flubendazole, fenbendazole, oxfendazole, or a pharmaceutically acceptable salt thereof where applicable, or a combination thereof.

## FIG.1

## FIG. 2

## FIG.3

FIG.4

FIG.5

## *FIG.6*

## *FIG.7*

## FIG.8

## FIG.9

## FIG.10

### FIG.11

### FIG.12

# SCHEME.1.

# SCHEME.2.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 149 510 (ELI LILLY AND CO.)(24-07-1985) --- | | A 61 M  31/00<br>A 61 D   7/00 |
| A | WO-A-8 200 094 (LABY)(12-01-1982) ----- | | |

TECHNICAL FIELDS
SEARCHED (Int. Cl.5)

A 61 M
A 61 D

The present search report has been drawn up for all claims

| Place of search | Date of completion of the search | Examiner |
|---|---|---|
| THE HAGUE | 21-08-1990 | VANRUNXT J.M.A. |